# FASCICULE DE BREVET EUROPEEN

(11) **EP 1 648 496 B1**
(45) Date de publication et mention de la délivrance du brevet: **22.02.2012**
(21) Numéro de dépôt: 04767620.0
(22) Date de dépôt: 08.07.2004
(51) Int. Cl.: A61K 38/36, A61K 38/37, A61K 47/18, A61K 47/12, A61L 2/00, C07K 14/75

(54) **PROCEDE DE STABILISATION D'UN CRYOPRECIPITE DE PROTEINES PLASMATIQUES DESTINE A ETRE SOUMIS A UN TRAITEMENT THERMIQUE D'INACTIVATION VIRALE**
VERFAHREN ZUR STABILISIERUNG EINES KRYOPRÄZIPITATS VON PLASMATISCHEN PROTEINEN FÜR EINE WÄRMEBEHANDLUNG ZUR VIRALEN INAKTIVIERUNG
METHOD FOR STABILIZING A CRYOPRECIPITATE OF PLASMATIC PROTEINS FOR BEING SUBJECTED TO A VIRAL INACTIVATION THERMAL TREATMENT

(30) Priorité: 09.07.2003 FR 0308403
(43) Date de publication de la demande: 26.04.2006
(73) Titulaire: Laboratoire Français du Fractionnement et des Biotechnologies, 91940 Les Ulis (FR)
(72) Inventeur: BARDAT, Annie, F-91470 Limours (FR); BEGIN, Edith, F-91940 Les Ulis (FR)
(74) Mandataire: Hirsch & Associés
(86) Numéro de dépôt international: PCT/FR2004/001788
(87) Numéro de publication internationale: WO 2005/004901

(56) Documents cités:
- EP-A- 1 197 221
- WO-A-99/10011
- US-A- 4 470 968
- US-A- 4 623 717
- US-A- 4 650 678
- US-A- 4 992 419
- US-A- 5 399 670
- US-A- 5 831 027
- US-A- 5 919 443
- US-A1- 2001 049 361
- WINKELMAN L ET AL: "SEVERE HEAT TREATMENT OF LYOPHILISED COAGULATION FACTORS" CURRENT STUDIES IN HEMATOLOGY AND BLOOD TRANSFUSION, KARGER, BASEL, CH, no. 56, 1989, pages 55-69, XP009028964 ISSN: 0258-0330 cité dans la demande
- MARGOLIS J ET AL: "STABILISING EFFECT OF AMINOACIDS ON FACTOR VIII IN LYOPHILISED CRYOPRECIPITATE" LANCET THE, LANCET LIMITED. LONDON, GB, 8 décembre 1984 (1984-12-08), page 1345, XP009028968 ISSN: 0140-6736 cité dans la demande
- KYTE J ET AL: "A SIMPLE METHOD FOR DISPLAYING THE HYDROPATHIC CHARACTER OF A PROTEIN" JOURNAL OF MOLECULAR BIOLOGY, LONDON, GB, vol. 157, no. 1, 5 mai 1982 (1982-05-05), pages 105-132, XP000609503 ISSN: 0022-2836 cité dans la demande

## Description

La présente invention concerne un procédé d'obtention de protéines cryoprécipitables issues du plasma sanguin, en général par cryoprécipitation ou par précipitation alcoolique à froid, faisant intervenir une étape de lyophilisation et une étape ultérieure d'inactivation virale par traitement thermique du lyophilisat, comprenant une étape d'ajout d'une formulation stabilisante et solubilisante permettant une lyophilisation de compositions liquides dedites protéines et une remise en solution aisée des formes lyophilisées après traitement thermique d'inactivation virale. Dans le cadre de l'invention, le terme "protéine" recouvre la protéine en tant que telle et également les concentrés et les fractions, contenant une telle protéine, notamment à usage thérapeutique, seule ou en mélange avec d'autres telles protéines. Ces concentrés et fractions sont obtenus par des méthodes de fractionnement du plasma humain ou animal connues dans l'art antérieur. Egalement, la locution "composition liquide de protéines cryoprécipitables" signifie une composition liquide comprenant au moins une protéine caractérisée par son insolubilité à froid lors de la décongélation d'un plasma humain ou animal congelé, ou bien par son insolubilité lors d'une précipitation par l'ajout au plasma d'un solvant organique, tel que l'éthanol, à froid.

L'utilisation de produits thérapeutiques issus du plasma humain, tels que les facteurs de la coagulation, à des fins thérapeutiques, notamment dans le cas de troubles hémorragiques héréditaires tels que l'hémophilie, peut être grandement compromise par le risque considérable que présente pour le patient hémophile la présence de virus dans les produits sanguins. Malgré la sélection rigoureuse de donneurs individuels, il persiste un risque de transmission de divers virus notamment ceux de l'hépatite et du SIDA et des virus inconnus à ce jour et pouvant se révéler transmissibles par les produits sanguins.

Par conséquent, la transmission virale doit être évitée par des traitements adéquats des différentes fractions purifiées issues du plasma des donneurs et destinées à un usage thérapeutique. A ce titre, diverses méthodes d'inactivation ou d'élimination virale appliquées à diverses fractions protéiniques issues du plasma sanguin sont bien connues. On peut citer, par exemple, leurs traitements par solvant-détergent, ultrafiltration et nanofiltration, par pasteurisation ou par chauffage prolongé. Dans le cas du traitement par chauffage prolongé, celui-ci ne s'applique habituellement qu'à des fractions de protéines du plasma préalablement lyophilisées tout en nécessitant des températures de chauffage d'au moins 70°C sur des laps de temps compris entre 50 et 100 heures pour une inactivation virale optimale. Cependant, sous de telles conditions sévères de traitement thermique, les protéines plasmatiques, fragiles et thermolabiles, subissent des dégradations, ce qui conduit à des diminutions importantes de leurs fonctions biologiques.

Afin de remédier à cet inconvénient, des excipients protecteurs et stabilisants de protéines plasmatiques sont préalablement ajoutés à des compositions protéiniques liquides avant lyophilisation afin de répondre à un double objectif conjoint. Le premier objectif répond à la nécessité d'une stabilisation d'une part des protéines considérées au cours de la lyophilisation et, d'autre part, des protéines lyophilisées lors du stockage et, le second objectif, correspond au besoin d'une protection des protéines lyophilisées au cours du traitement thermique d'inactivation virale.

Le brevet EP 0 094 611 décrit une méthode de chauffage de fractions protéiniques du plasma lyophilisées, le Facteur VIII ou le fibrinogène, consistant à soumettre le produit sec à une température de chauffage de 60°C pendant 72 à 96 heures. Ce brevet ne fait mention d'aucune composition particulière d'excipients stabilisants au cours du traitement thermique.

Le brevet canadien 1 260 389 mentionne l'incorporation d'excipients, tels que des anions non polaires de masse moléculaire supérieure à 80, des sucres, des sucres réducteurs et des acides aminés notamment, dans des compositions liquides de protéines du plasma préalablement à la lyophilisation afin de leur conférer une stabilisation au chauffage à sec pendant environ 72 heures à 68°C. Cependant, l'association de sucres réducteurs avec des acides aminés conduit à des composés de Maillard dont les propriétés ne sont pas en faveur d'une bonne innocuité des protéines traitées (activité, immunogénécité, allergies etc.). Ce traitement nécessite d'opérer sous vide ou sous atmosphère inerte.

La plupart des excipients stabilisants peuvent se révéler protecteurs de fractions de protéines plasmatiques au cours du traitement thermique à sec de celles-ci, à des températures de l'ordre de 60°C-68°C pendant 30 à 96 heures (P. Thomas, British Journal of Haematology, 70, 1998, 393-395 et J.A. Levy et al., The Lancet, June 22, 1985, 1456-1457). Toutefois, malgré une diminution du titre viral après un tel traitement dans ces conditions, il a été démontré que des infections telles que HIV, HBV, HBC et parvovirus B19 pouvaient néanmoins être transmises (P. Thomas cité ci-dessous). Pour leur élimination efficace, il a été proposé de chauffer les fractions de protéines plasmatiques lyophilisées à des températures plus élevées. Ainsi, S.J. Skidmore et al. (Journal of Medical Virology, 30, 1990, 50-52) ont montré qu'un traitement thermique de concentrés de Facteur VIII lyophilisés à une température de 80°C pendant 72 heures évite la transmission du virus HCV non-A et non-B. Le brevet US 5 831 027 décrit ainsi un procédé de traitement thermique d'une protéine lyophilisée issue du cryoprécipité de plasma sanguin, le fibrinogène, à une température de 80°C pendant 72 heures permettant ainsi l'obtention du fibrinogène exempt d'éventuels virus tels que ceux de HBV, HBC ou le parvovirus B19. Les excipients stabilisants ajoutés pour protéger la composition de fibrinogène à la fois au cours de la lyophilisation et pendant le traitement thermique d'inactivation virale, comprennent le saccharose et/ou un acide aminé (arginine), le tampon Tris et le citrate de sodium. L. Wilkelman et al. (Virus Inactivation in Plasma Products., Curr.Stud Hematol Blood Transfus., Basel, Karger, 1989, N°56, 55-69) montrent également la nécessité d'ajout d'excipients au Facteur VIII préalablement à la lyophilisation et à un traitement thermique d'inactivation virale à 80°C pendant 72 heures. Les excipients décrits sont : NaCl, citrate de sodium, Tris, CaCl₂ et saccharose.

Par ailleurs, étant donné que les différentes protéines issues du fractionnement du plasma, ayant subi une lyophilisation et un traitement thermique d'inactivation virale, nécessitent une reconstitution dans un milieu adéquat avant leur utilisation clinique, celle-ci doit pouvoir être facilement mise en oeuvre sur un laps de temps relativement court selon les exigences préconisées par la Phamacopée Européenne. A cet égard, des études sur la stabilité thermique du Facteur VIII dans un cryoprécipité lyophilisé (J. Margolis et al., The lancet, December 8, 1984, 1345) contenant, préalablement à la lyophilisation, un mélange d'acides aminés naturels approprié à une alimentation parentérale, la Synthamin 17% (Travenol Laboratories Ltd.), ont montré qu'un traitement à 80°C pendant 16 heures, entraînait non seulement une dégradation du Facteur VIII telle que son activité était nulle mais également une impossibilité de redissoudre le cryoprécipité après les opérations considérées. Le brevet US 5 399 670 décrit un procédé pour faciliter la solubilisation ou la reconstitution des compositions de complexe de Facteur VIII lyophilisées avec de l'eau purifiée pour injection, comprenant une étape d'ajout d'arginine à une solution de Facteur VIII préalablement à sa lyophilisation. Ce brevet ne mentionne pas de traitement thermique d'inactivation virale. Il peut également être prévu, selon ce brevet, l'ajout d'histidine et d'albumine. Les excipients stabilisants mentionnés dans le brevet US 5 831 027 cité précédemment, sont également destinés à favoriser la dissolution du fibrinogène lyophilisé dans l'eau pure, préalablement à son usage thérapeutique.

Toutefois, le choix d'une formulation stabilisante est dicté par la spécificité des protéines plasmatiques. Ainsi, en référence à l'article de N. Heimburger et al. (Virus Inactivation in Plasma Products., Curr Stud Hematol Blood Transfus., Basel, Karger, 1989, N°56, 23-33), on considère habituellement qu'une formulation stabilisante spécifique ne peut convenir qu'à une fraction protéinique aux principes actifs donnés. Une difficulté supplémentaire apparaît dans le cas où des fractions protéiniques plus complexes sont envisagées, notamment celles où sont considérées toutes les protéines de la coagulation et de l'hémostase issues d'un fractionnement du plasma. Par ailleurs, les hydrates de carbone, notamment le saccharose, peuvent être utilisés efficacement comme excipients de stabilisation et de redissolution de fractions protéiniques du plasma, lorsqu'on se propose de lyophiliser les fractions considérées puis de les traiter par la chaleur à sec, bien qu'ayant un effet ralentisseur sur l'inactivation virale (N. Heimburger *et al.* cité ci-dessus). Par conséquent, les fractions protéiniques ainsi traitées peuvent ne pas être totalement exemptes de virus et leur utilisation sur le plan clinique s'en trouve restreinte. En outre, certains hydrates de carbone, comme le maltose ou le saccharose, ne peuvent être utilisés sans risques chez des sujets présentant des insuffisances rénales et/ou souffrant du diabète.

Par conséquent, compte tenu du besoin médical existant pour certaines protéines responsables de la coagulation et de l'hémostase, en particulier les protéines cryoprécipitables, la Demanderesse a cherché à mettre au point une formulation simple, exempte d'hydrates de carbone et de tampon Tris, compatible avec un usage thérapeutique, qui, ajoutée à des compositions liquides de protéines cryoprécipitables, confère une bonne protection de tous les principes actifs considérés pendant et après la lyophilisation de celles-ci, d'une part, et, d'autre part, contre les chocs thermiques nécessaires à la destruction des virus et qui permet, en même temps, un temps de remise en solution réduit des formes lyophilisées de ces protéines.

A cette fin, considérant que l'ajout d'arginine à des compositions liquides de protéines cryoprécipitables offre un effet protecteur pendant et après la lyophilisation de celles-ci, tout en permettant la solubilisation de leurs formes lyophilisées mais sans assurer leur stabilité au traitement thermique d'inactivation virale, la Demanderesse a cherché différents composés, seuls ou en mélange, dont l'ajout à l'arginine offrirait une protection contre la dénaturation thermique. Ainsi, la Demanderesse a trouvé de façon surprenante que l'addition à l'arginine, acide aminé très hydrophile, d'au moins un acide aminé hydrophobe, de préférence choisi parmi les plus hydrophobes selon Kyte et al. (J. Mol. Biol., 157, 105-132, 1982), et de citrate trisodique permettait la stabilisation des protéines cryoprécipitables pendant et après la lyophilisation, tout en améliorant de façon notable la solubilisation des formes lyophilisées après le traitement thermique d'inactivation virale.

Par conséquent, l'invention concerne un procédé d'obtention de protéines cryoprécipitables, comprenant une étape d'inactivation virale par traitement thermique d'un lyophilisat desdites protéines, caractérisé en ce qu'il comprend, avant la mise des protéines sous la forme de lyophilisat, une étape initiale d'ajout, auxdites protéines, d'une formulation stabilisante et solubilisante comprenant un mélange d'arginine, d'au moins un acide aminé hydrophobe et de citrate trisodique.

Ainsi, l'ajout de la formulation stabilisante et solubilisante dans le procédé selon l'invention permet de conserver aux protéines cryoprécipitables à partir du plasma sanguin une activité biologique satisfaisante après lyophilisation et traitement thermique d'inactivation virale, même si celui-ci est sévère, et de présenter un temps de remise en solution réduit tout en préservant un caractère de limpidité à la solution du lyophilisat reconstitué. Cette formulation présente en outre l'avantage d'être simple, universelle et de pouvoir être aisément mise en oeuvre sur le plan industriel avec des gains de temps appréciables.

De préférence, la formulation stabilisante et solubilisante utilisée dans le procédé de l'invention est constituée du seul mélange d'arginine, d'au moins un acide aminé hydrophobe et de citrate trisodique. Une telle formulation exclusivement constituée de ces trois composés, présente particulièrement l'avantage d'associer une réduction des durées et des coûts de préparation à l'échelle industrielle grâce à la présence d'un nombre minimal mais efficace d'adjuvants.

Tout acide aminé hydrophobe (selon Kyte *et al.* cité ci-dessus), tel que la valine et la phénylalanine, convient dans le cadre de l'invention mais avantageusement le choix se porte sur la leucine, l'iso-leucine ou un mélange des deux.

La formulation stabilisante et solubilisante comprend également du citrate trisodique qui permet, d'une part, d'ajuster le pH des compositions liquides de protéines cryoprécipitables avant les traitements mentionnés ci-dessus et, d'autre part, d'accroître l'effet protecteur de celle-ci à condition d'en ajuster la concentration.

Enfin, la formulation stabilisante et solubilisante peut en outre être additionnée de glycine et/ou de lysine.

Elle peut également, en tant que de besoin, être additionnée d'adjuvants stabilisants connus dans la technique.

Dans le contexte de l'invention, on peut ajouter la formulation stabilisante et solubilisante comprenant le mélange des trois composés d'intérêt à une composition liquide de protéines cryoprécipitables, ou les protéines cryoprécipitables peuvent être dissoutes dans une solution aqueuse de la formulation comprenant le mélange des trois composés d'intérêt.

Les concentrations des différents additifs envisagés pour la formulation stabilisante seront choisies par l'homme du métier de façon à obtenir l'effet de stabilisation voulu. Avantageusement, les concentrations en chacun des additifs, par litre de compositions liquides de protéines cryoprécipitables, sont les suivantes :
- arginine, de 25 à 50 g/l et de préférence de 35 à 45 g/l (en référence au brevet US 5 399 670);
- citrate trisodique, de 0,5 à environ 12 g/l ;
- leucine, isoleucine ou leur mélange, de 5 à 15 g/l et de préférence de 9 à 11 g/l ; et
- glycine et/ou lysine, chacune de 1 à 5 g/l et de préférence chacune de 1,5 à 2,5 g/l.

Dans le cadre de l'invention, la lyophilisation de compositions liquides de protéines préalablement congelées est effectuée selon des méthodes classiques utilisant des dispositifs habituels, selon des conditions de mise en oeuvre connues de l'homme du métier. Avantageusement, la lyophilisation est effectuée à des températures comprises entre -40°C et -30°C pendant environ 48 heures.

Le traitement thermique d'inactivation virale est effectué de façon à inactiver efficacement les virus. Il est mis en oeuvre de préférence à des températures comprises entre 80°C et 90°C pendant 72 heures.

Bien que le traitement thermique d'inactivation virale permette l'obtention d'un lyophilisat exempt de virus, le procédé peut comprendre, préalablement à l'étape d'ajout de la formulation stabilisante et solubilisante à une composition liquide de protéines cryoprécipitables, au moins une étape supplémentaire d'inactivation et/ou d'élimination des virus de ladite composition liquide par solvant-détergent et/ou par nanofiltration, par exemple sur des filtres de 35 nm, pour assurer au final une inactivation et une élimination totales et complètes des virus.

Ainsi, la mise en oeuvre du procédé conduit à des protéines cryoprécipitables lyophilisées et viralement inactivées, qui, une fois reconstituées dans un milieu liquide compatible sur le plan pharmaceutique, tel que l'eau pure pour injection, peuvent être injectées directement à un patient. Cette qualité thérapeutique des protéines cryoprécipitables est obtenue grâce à la formulation stabilisante et solubilisante qui rend possible tous les traitements mentionnés précédemment, notamment les traitements d'inactivation et d'élimination des virus, tout en conservant l'activité biologique de ces protéines cryoprécipitables traitées et les caractéristiques de dissolution du lyophilisat.

La formulation stabilisante et solubilisante utilisée selon le procédé de l'invention s'applique aux protéines cryoprécipitables, telles que le Facteur VIII, le Facteur von Willebrand, le Facteur XIII, le fibrinogène et la fibronectine, obtenues par des méthodes de fractionnement du plasma sanguin connues de l'homme du métier. La formulation stabilisante et solubilisante s'applique également aux différents concentrés de protéines semi-purifiés, obtenus par exemple par extraction/solubilisation en tampon Tris et adsorption sur gel d'alumine, tel que décrit par Wickerhauser et al. (Vox Sang., 35, 18-31, 1978). Un concentré ainsi obtenu, enrichi en Facteur VIII et Facteur von Willebrand, peut être chauffé selon les conditions décrites dans le brevet EP 0 094 611.

Cette formulation est également appropriée à la stabilisation et à la solubilisation des protéines purifiées ou des fractions enrichies en chacun des facteurs de la coagulation telles qu'obtenues notamment après la mise en oeuvre de méthodes chromatographiques décrites, par exemple, dans le brevet EP 0 359 593, ayant été ensuite lyophilisées et ayant subi un traitement thermique d'inactivation virale. Par ailleurs, cette formulation est appropriée à la stabilisation du fibrinogène purifié obtenu à partir du cryoprécipité de plasma ou du plasma par précipitation alcoolique à froid, telle que décrite par Kistler et al. (Vox Sang., 7, 1962, 414-424). Il convient de noter que dans le contexte de l'invention, lors de la purification du fibrinogène à partir d'un cryoprécipité par toute technique de fractionnement connue de l'homme du métier, celui-ci est toujours accompagné d'une faible teneur en Facteur XIII (FXIII), non rédhibitoire pour son activité thérapeutique.

Ce procédé est particulièrement avantageux parce qu'il peut être appliqué à l'ensemble des protéines cryoprécipitables ou à au moins une protéine choisie parmi celles-ci et, en particulier, choisie parmi le Facteur VIII, le Facteur von Willebrand, le Facteur XIII, le fibrinogène et la fibronectine.

L'invention se rapporte également aux concentrés d'au moins une protéine cryoprécipitable, notamment à usage thérapeutique, comprenant la formulation stabilisante et solubilisante selon le procédé de l'invention.

Enfin, l'invention concerne une formulation stabilisante et solubilisante pour les protéines cryoprécipitables destinées à être soumises à une lyophilisation et à un traitement thermique d'inactivation virale comprenant un mélange d'arginine, d'au moins un acide aminé hydrophobe et de citrate trisodique. De préférence, la formulation stabilisante et solubilisante est constituée dudit mélange d'arginine, d'au moins un acide aminé hydrophobe et de citrate trisodique.

Les exemples suivants illustrent l'invention sans toutefois en limiter la portée.

### Exemple 1

Un cryoprécipité, constitué en grande majorité de Facteur VIII, de facteur von Willebrand, de Facteur XIII, de fibrinogène et de fibronectine, a été remis en solution dans une formulation stabilisante et solubilisante de l'invention comprenant le mélange de composés donnés au Tableau 1 (Solution A). La concentration en protéines est d'environ 15 g/l.

**Tableau 1 : composés et leurs concentrations (Solution A)**

| Composés | Concentration (g/l) |
|---|---|
| Arginine | 40 |
| Iso-leucine | 10 |
| Citrate trisodique | 2,5 |
| Lysine | 2 |
| Glycine | 2 |

Après homogénéisation du mélange, la solution ainsi obtenue est filtrée sur des filtres de 0,45 µm et 5 ml sont prélevés et placés dans un flacon. La solution subit ensuite une lyophilisation à -30°C pendant 48 heures. On procède de façon identique avec une solution de référence comprenant le même cryoprécipité que le précédent mais qui a été remis en solution dans une formulation standard comprenant un mélange respectif de Tris (2,4 g/l), de citrate trisodique (5,88 g/l) et de NaCl (1,16 g/l) (Solution B).

Après la lyophilisation, les deux lyophilisats de cryoprécipité obtenus, à savoir l'un comprenant la formulation de l'invention et l'autre la formulation standard, sont remis en solution dans 5 ml d'eau pure pour injection (respectivement dénommées Solution A' et Solution B'). On procède alors à des expérimentations destinées à apprécier l'aptitude des formulations standard et celle de l'invention à protéger ou à stabiliser l'ensemble des protéines considérées durant la lyophilisation, conjointement à solubiliser les formes lyophilisées obtenues. A cet effet, on évalue, pour chacune des Solutions A' et B', les trois paramètres suivants : l'aspect du lyophilisat avant redissolution, le temps de redissolution du lyophilisat dans l'eau purifiée pour injection et la turbidité de la solution ainsi obtenue, conjointement aux activités et aux quantités des différentes protéines par des méthodes connues de l'homme du métier. Les différents résultats des mesures sont présentés dans le Tableau 2 dans lequel les unités de volume sont relatives à la solution du produit lyophilisé reconstitué par 5 ml d'eau purifiée pour injection.

**Tableau 2**

| | Solution A' | Solution B' |
|---|---|---|
| Aspect du lyophilisat sec | jaunâtre | jaunâtre, lyophilisat rétracté |
| Temps de redissolution (min) | 6,58 | 10,72 |
| Turbidité (NTU*) | 18,1 | 29 |
| Fibrinogène coagulable (g/l) | 12,6 | 14,5 |
| Fibrinogène pondéral (g/l) | 11,6 | 11,2 |
| Activité en Facteur VIII (UI/ml) | 6,7 | 6,3 |
| Activité en Facteur von Willebrand (FvW:RCo ; UI/ml) | 8,1 | 8,1 |
| Activité antigène en Facteur XIII (UI/ml) | 1,51 | 1,24 |
| Fibronectine (mq/ml) | 5,85 | 5,52 |

| | | |
|---|---|---|
| *NTU : Normalized Turbidity Units | | |

Les résultats obtenus montrent tout d'abord que l'ajout d'une formulation de l'invention (Solution A) aux protéines du cryoprécipité, par rapport à la formulation standard (Solution B), permet de réduire sensiblement le temps de redissolution du lyophilisat, de l'ordre de 40%. On observe également que la formulation A donne de meilleurs résultats sur le plan de la turbidité ce qui indique une présence diminuée, par rapport à la solution B, en produits de dégradation insolubles dans l'eau. Dans les deux cas, les Solutions A et B restituent globalement les mêmes quantités et activités des protéines considérées après lyophilisation.

### Exemple 2

Les deux lyophilisats de protéines du cryoprécipité précédents, à savoir l'un comprenant la formulation de l'invention et l'autre la formulation standard, sont chauffés à sec pendant 72 heures à 80°C. Le lyophilisat chauffé des protéines du cryoprécipité comprenant une formulation de l'invention (Solution A) est remis en solution dans 5 ml d'eau pure pour injection (Solution A"). On observe que le lyophilisat chauffé des protéines du cryoprécipité comprenant la formulation standard (Solution B) ne peut être remis en solution. Cette impossibilité de redissolution s'explique par la présence de protéines dénaturées par la chaleur et insolubles, ce qui démontre que la solution B ne stabilise pas les protéines au cours du traitement thermique. Comme pour l'Exemple 1, on procède toutefois aux mêmes expérimentations destinées à apprécier l'aptitude de la formulation de l'invention à stabiliser et à solubiliser conjointement l'ensemble des protéines considérées après le traitement thermique d'inactivation virale effectué sur leurs formes lyophilisées de l'Exemple 1. Les différents résultats des mesures sont présentés dans le Tableau 3 dans lequel les unités de volume sont relatives à la solution du produit lyophilisé reconstitué par 5 ml d'eau purifiée pour injection.

**Tableau 3**

| | Solution A" |
|---|---|
| Aspect du lyophilisat sec | jaune citron |
| Temps de redissolution (min) | 3,73 |
| Turbidité (NTU*) | 18,3 |
| Fibrinogène coagulable (g/l) | 13,3 |
| Fibrinogène pondéral (g/l) | 11,7 |
| Activité en Facteur VIII (UI/ml) | 5,6 |
| Activité en Facteur von Willebrand (FvW:RCo ; UI/ml) | 6,0 |
| Activité antigène en Facteur XIII (UI/ml) | 1,86 |
| Fibronectine (mg/ml) | 5,93 |

| | |
|---|---|
| *NTU : Normalized Turbidity Units | |

La comparaison des résultats des mesures obtenus pour les Solutions A' et A", extraits respectivement des Tableaux 1 et 2, montre de façon surprenante que la Solution A, formulation selon l'invention, permet une réduction très importante, d'environ 50%, du temps nécessaire à la redissolution des protéines traitées thermiquement par rapport à celui obtenu après lyophilisation, sans pertes notables de leurs fonctions biologiques.

### Exemple 3

Un lot de fibrinogène, ayant été isolé et purifié à partir d'un cryoprécipité par la méthode de Kistler *et al.*, a été mis en solution à raison de 15 g/l dans une formulation témoin constituée d'un mélange de citrate trisodique (2,5 g/l), de lysine (2 g/l) et de glycine (2 g/l) (Solution C). On obtient ainsi une solution concentrée en fibrinogène. A cette solution, on ajoute divers acides aminés afin d'étudier leur influence sur le temps de redissolution du lyophilisat de fibrinogène avant et après chauffage. Les solutions obtenues, ainsi que les concentrations en acides aminés, sont indiquées au Tableau 4.

**Tableau 4**

| Solution | Acide aminé (g/l) |
|---|---|
| C | - |
| C1 | C + valine (5 g/l) |
| C2 | C + leucine (5 g/l) |
| C3 | C + arginine (10 g/l) |
| C4 | C + isoleucine (10 g/l) |
| C5 | C + Isoleucine (10 g/l) + arginine (10 g/l |

Les differentes solutions (Solutions C à C5) sont ensuite filtrées et 10 ml de chaque solution sont soumis à une lyophilisation et au traitement thermique selon l'Exemple 1. Les lyophilisats respectifs sont repris dans 10 ml d'eau pure pour injection et on mesure le temps nécessaire pour une redissolution totale des lyophilisats. Les résultats sont présentés au Tableau 5.

**Tableau 5**

| Solution | Temps de redissolution avant chauffage (min) | Temps de redissolution après chauffage (min) |
|---|---|---|
| C | 32,66 | 61,50 |
| C1 | 15,22 | 8,05 |
| C2 | 16,3 | 22,93 |
| C3 | 6,0 | 12,69 |
| C4 | 11,25 | 10,75 |
| C5 | 5,7 | 4,85 |

Les résultats montrent bien que la Solution C5 selon l'invention offre le temps de redissolution le plus court.

Afin de montrer également l'aptitude des solutions considérées à stabiliser le fibrinogène au cours de la lyophilisation et du chauffage des formes sèches, en fonction de la nature de l'acide aminé ajouté, on procède à des mesures de turbidité des solutions précédentes reconstituées. Le Tableau 6 présente les mesures de la turbidité avant chauffage des lyophilisats des Solutions C à C5, de même qu'après chauffage de celles-ci.

**Tableau 6**

| Solution | Turbidité avant chauffage (*NTU) | Turbidité après chauffage (*NTU) | Accroissement (%) |
|---|---|---|---|
| C | 24,25 | 34,65 | 42,9 |
| C1 | 18,04 | 22,83 | 26,6 |
| C2 | 18,48 | 24,68 | 35,6 |
| C3 | 15,44 | 18,80 | 21,8 |
| C4 | 13,02 | 16,06 | 23,3 |
| C5 | 10,98 | 11,88 | 8,2 |

| | | | |
|---|---|---|---|
| (*NTU) : Normalized Turbidity Units | | | |

Les résultats indiquent de manière indubitable qu'une formulation de l'invention (Solution C5) présente la plus faible différence de turbidité des solutions reconstituées, avant et après chauffage, ce qui se traduit par un accroissement de seulement 8,2%, par rapport à la Solution C dont l'accroissement est de 42,9%.

### Exemple 4

Les Solutions C, C3 à C5 de l'Exemple 3, contenant un autre lot de fibrinogène, sont lyophilisées et chauffées à 80°C pendant 72 heures. Les lyophilisats correspondant sont ensuite repris dans 10 ml d'eau pure pour injection et on mesure les paramètres suivants : le temps de redissolution, la quantité en multimères non solubles, la turbidité, et la filtrabilité des solutions reconstituées par des méthodes connues de l'homme du métier. En particulier, la filtrabilité permet d'apprécier le degré de dénaturation d'une protéine, le fibrinogène dans le cas présent, sans toutefois définir le ou les facteur(s) de dénaturation qui peuvent être proportionnels à la quantité en particules, fibrilles ou multimères. De même, la teneur en multimères est également proportionnelle au degré de dénaturation du fibrinogène et est mesurée par électrophorèse (SDS Page). L'essai de filtrabilité consiste à mesurer le volume filtré récupéré d'une solution à travers un filtre à porosité suffisante pour assurer la stérilisation de la solution, soit de 0,20 ± 0,02 µm et de 25 mm de diamètre au moyen d'une seringue contenant 10 ml de la solution à étudier. Le volume filtré récupéré traduit l'importance du colmatage du filtre par les produits de dégradation. Ainsi, plus le volume récupéré est grand, moins le fibrinogène est dégradé. Les résultats des différentes mesures sont présentés dans le Tableau 7.

**Tableau 7**

| Solution | Filtrabilité (ml) | Temps de redissolution (min) | Quantité en multimères (%) | Turbidité (*NTU) |
|---|---|---|---|---|
| C | 7 | 20 | 10 | 26 |
| C3 | 10 | 20 | 10 | 21 |
| C4 | 10 | 10 | 5 | 11 |
| C5 | 10 | 4 | < 3 | 11 |

| | | | | |
|---|---|---|---|---|
| (*NTU) : Normalized Turbidity Units | | | | |

Les quatre paramètres analysés ci-dessus indiquent qu'une formulation de la présente invention (Solution C5) est particulièrement adaptée à la stabilisation et à la redissolution du lyophilisat de fibrinogène chauffé. Le lyophilisat de fibrinogène reconstitué présente une filtrabilité rapportée à la surface du filtre d'environ 2 ml/cm².

Afin de démontrer les pouvoirs stabilisant et solubilisant de la Solution C5 selon l'invention vis-à-vis du fibrinogène même à des conditions sévères de traitement thermique, les Solutions C3 et C5 contenant un autre lot de fibrinogène, ont été lyophilisées et chauffées à 90°C pendant 72 heures. Hormis l'étude des quatre paramètres ci-dessus, on a procédé à des essais supplémentaires consistant à des mesures du taux de produits de dégradation du fibrinogène (PDF). Dans le cadre de cet exemple, les PDF (µg/ml) représentent des peptides de tailles diverses engendrés lors d'une dénaturation du fibrinogène. Plus cette valeur est importante, plus celui-ci apparaît dégradé et est susceptible de former par exemple des caillots. Les résultats des différentes mesures sont présentés dans le Tableau 8.

**Tableau 8**

| Solution | Filtrabilité (ml) | Temps de redissolution (min) | Quantité en multimères (%) | Turbidité (*NTU | PDF (µg/ml) |
|---|---|---|---|---|---|
| C3 | ≤ 6 | 10 | 10 | 16 | 750 à 1250 |
| C5 | 10 | 10 | 10 | 11 | 625 à 750 |

Les résultats ci-dessus démontrent que malgré des conditions de chauffage très sévères, la formulation selon l'invention (Solution C5) permet encore de protéger et de solubiliser le fibrinogène après une lyophilisation et un traitement thermique de 90°C pendant 72 heures, ce qui est révélé par de bonnes valeurs des paramètres étudiés. Le lyophilisat de fibrinogène reconstitué présente également une filtrabilité rapportée à la surface du filtre d'environ 2 ml/cm2.

### Exemple 5

Cet exemple traite de l'influence de la concentration en citrate trisodique contenu dans une formulation de l'invention (Solution A) sur la stabilisation et la solubilisation d'une solution de fibrinogène destinée à être lyophilisée et chauffée à 80°C pendant 72 heures. Un lot de fibrinogène, obtenu à partir d'un cryoprécipité, a été mis en solution à raison de 15 g/l et homogénéisé dans la Solution A dans laquelle on a fait ensuite varier la concentration en citrate trisodique d'une solution à l'autre. On a obtenu quatre solutions, notées respectivement Solutions A1, A2, A3 et A4, contenant respectivement 0,5 g/l, 1 g/l, 2 g/l et 11,2 g/l en citrate trisodique. Ces solutions sont ensuite filtrées comme précisé à l'Exemple 1 et 5 ml de chacune des solutions sont prélevés et placés dans un flacon. Les quatres solutions précédentes contenant le fibrinogène ont subi une lyophilisation et un traitement thermique indiqué à l'Exemple 1. Les quatre lyophilisats de fibrinogène non chauffés d'une part, et, d'autre part, chauffés sont ensuite remis en solution dans 5 ml d'eau pure pour injection pour donner les quatre solutions A1, A2, A3 et A4 précédentes. On procède à des expérimentations destinées à apprécier l'influence de la concentration en citrate trisodique de la formulation de l'invention sur l'aptitude de celle-ci à protéger le fibrinogène durant la lyophilisation et à le solubiliser après le chauffage des formes lyophilisées, ceci par rapport à ces solutions n'ayant subi aucune des opérations précédentes (solutions témoins correspondantes). A cet effet, on mesure, pour chacune des Solutions A1, A2, A3 et A4, les paramètres suivants : le temps de redissolution du lyophilisat dans l'eau pure pour injection, la turbidité de la solution ainsi obtenue, les quantités en multimères insolubles, en fibrinogène pondéral et coagulant par des méthodes connues de l'homme du métier. Les différents résultats des mesures sont présentés dans le Tableau 9 dans lequel les unités de volume sont relatives à la solution du produit lyophilisé reconstitué par 5 ml d'eau purifiée pour injection.

**Tableau 9**

| | Avant lyophilisation | | | | Après lyophilisation | | | | Après chauffage (80°C 72 h) | | | |
|---|---|---|---|---|---|---|---|---|---|---|---|---|
| | A1 | A2 | A3 | A4 | A1 | A2 | A3 | A4 | A1 | A2 | A3 | A4 |
| Quantité en multimères (%) | 8,3 | 6,6 | 7,3 | 7,5 | 6,9 | 5,6 | 5,8 | 4,2 | 6,3 | 6,2 | 5,9 | 5,2 |
| Turbidité *NTU | 11,0 | 11,0 | 10,9 | 10,1 | 11,3 | 11,1 | 10,9 | 10,1 | 11,6 | 11,3 | 11,2 | 10,3 |
| Fibrinogène coagulable (g/l) | 17,3 | 17,2 | 17,3 | 16,3 | 14,9 | 14,3 | 14,6 | 14,2 | 14,9 | 14,9 | 15,2 | 15,2 |
| Fibrinogène pondéral (g/l) | 16,6 | 16,1 | 16,0 | 15,8 | 16,2 | 16,1 | 16,0 | 15,2 | 16,7 | 15,7 | 16,9 | 16,1 |
| Temps de redissolution (min) | - | - | - | - | 5,50 | 10,87 | 6,37 | 10,03 | 10,35 | 9,83 | 11,55 | 9,95 |

| | | | | | | | | | | | | |
|---|---|---|---|---|---|---|---|---|---|---|---|---|
| *NTU : Normalized Turbidity Units | | | | | | | | | | | | |

Les résultats obtenus montrent que le choix d'une concentration dans la plage de valeurs de 0,5 à environ 12 g/l en citrate trisodique contenue dans une des formulations de l'invention ci-dessus non seulement permet une stabilisation satisfaisante du fibrinogène au cours de la lyophilisation et du traitement thermique, par rapport aux solutions témoins, mais permet également de conserver des temps de redissolution globalement identiques du fibrinogène lyophilisé par rapport à celui chauffé à sec.

### Exemple 6

Cet exemple traite de l'influence de la concentration en citrate trisodique contenu dans une formulation selon l'invention (Solution A) sur la stabilisation du Facteur XIII contenu dans une solution de fibrinogène destinée à être lyophilisée, d'une part, et, d'autre part, chauffée à 80°C pendant 72 heures. Ces deux protéines, ayant été obtenues à partir d'un cryoprécipité, ont été mises en solution à raison de 15 g/l (fibrinogène + Facteur XIII) et homogénéisées dans deux formulations selon l'invention (Exemple 1) contenant respectivement 2,5 g/l (Solution A) et 11,2 g/l (Solution A4) de citrate trisodique. Prélablement à la lyophilisation, les Solutions A et A4 ont subi les opérations décrites dans l'Exemple 1. Les deux lyophilisats de fibrinogène et de facteur XIII, d'une part, non chauffés et, d'autre part, chauffés sont ensuite remis en solution dans 5 ml d'eau purifiée pour injection pour donner les deux solutions A et A4 précédentes. On mesure respectivement l'activité en FXIII exprimées UI/ml (d'eau purifiée pour injection) et le FXIII-antigène en UI/ml ainsi que le rapport activité en FXIII:FXIII-antigène (noté R) selon des méthodes d'analyses classiques. Les différents résultats des mesures sont présentés dans le Tableau 10.

**Tableau 10**

| | Lyophilisat non chauffé | | | Lyophilisat chauffé | | |
|---|---|---|---|---|---|---|
| | Activité FXIII (UI/ml) | FXIII antigène (UI/ml) | R | Activité FXIII (UI/ml) | FXIII antigène (UI/ml) | R |
| Sol. A | 1,5 | 2,25 | 0,60 | 1,3 | 2,8 | 0,46 |
| Sol. A4 | 9,9 | 8,22 | 1,2 | 8,6 | 8,15 | 1,06 |

Les résultats montrent une légère baisse du rapport R pour chacun des lyophilisats lorsque ceux-ci ont été traités thermiquement, par rapport aux lyophilisats non chauffés. Par ailleurs, on note que la diminution du rapport R est moins importante lorsque la teneur en citrate est plus élevée. Ce tableau révèle en outre que lorsqu'on augmente la concentration en citrate d'une solution par rapport à une autre, en l'occurrence les Solutions A et A4, et qu'on les lyophilise, le rapport R augmente également. On observe le même phénomène lorsque les lyophilisats sont chauffés. Par conséquent, la concentration en citrate influe sur la stabilisation du FXIII ce qui est notamment démontré par les valeurs des différentes activités.

### Exemple 7

Dans cet exemple, on procède à la préparation des quatre solutions selon l'Exemple 5 qui sont reconstituées après lyophilisation et traitement thermique (80°C pendant 72 heures). Le Tableau 11 qui suit présente les mesures de l'activité en FXIII exprimée UI/ml (d'eau purifiée pour injection) et de FXIII-antigène en UI/ml ainsi que le rapport activité en FXIII:FXIII-antigène (noté R) en fonction des variations des concentrations en citrate dans les solutions.

**Tableau 11**

| Solution | Activité FXIII (UI/ml) | FXIII antigène (UI/ml) | R |
|---|---|---|---|
| A1 | 4,8 | 6,35 | 0,76 |
| A2 | 4,7 | 6,38 | 0,74 |
| A3 | 5,5 | 6,49 | 0,85 |
| A4 | 6,0 | 5,7 | 1,05 |

Les résultats obtenus montrent que plus la concentration en citrate trisodique contenue dans la formulation de l'invention est grande, moins le FXIII subit de dégradations.

## Revendications

1. Procédé d'obtention de protéines cryoprécipitables exemptes d'hydrates de carbone et de tampon tris, comprenant une étape d'inactivation virale par traitement thermique d'un lyophilisat desdites protéines, **caractérisé en ce qu'**il comprend, avant la mise des protéines sous la forme de lyophilisat, une étape initiale d'ajout, auxdites protéines, d'une formulation stabilisante et solubilisante, exempte d'hydrates de carbone et de tampon tris, comprenant un mélange d'arginine, d'au moins un acide aminé hydrophobe et de citrate trisodique.

2. Procédé selon la revendication 1, **caractérisé en ce que** la formulation est constituée dudit mélange d'arginine, d'au moins un acide aminé hydrophobe et de citrate trisodique.

3. Procédé selon la revendication 1 ou 2, **caractérisé en ce que** l'arginine est présente en une concentration comprise entre 25 et 50 g/l.

4. Procédé selon la revendication 3, **caractérisé en ce que** la concentration en arginine est comprise entre 35 et 45 g/l.

5. Procédé selon l'une quelconque des revendications 1 à 4, **caractérisé en ce que** le citrate trisodique est présent en une concentration comprise entre 0,5 et environ 12 g/l.

6. Procédé selon l'une quelconque des revendications 1 à 5, **caractérisé en ce que** l'acide aminé hydrophobe est la leucine, l'iso-leucine ou un mélange des deux.

7. Procédé selon la revendication 6, **caractérisé en ce que** la leucine, l'iso-leucine ou leur mélange est présent en une concentration comprise entre 5 et 15 g/l.

8. Procédé selon l'une des revendications 6 et 7, **caractérisé en ce que** la concentration en leucine ou iso-leucine ou leur mélange est comprise entre 9 et 11 g/l.

9. Procédé selon l'une quelconque des revendications 1 à 8, **caractérisé en ce que** la formulation est en outre additionnée de glycine et/ou de lysine.

10. Procédé selon la revendication 9, **caractérisé en ce que** la glycine et la lysine sont présentes chacune en une concentration comprise entre 1 et 5 g/l.

11. Procédé selon la revendication 10, **caractérisé en ce que** chacune des concentrations en glycine et en lysine est comprise entre 1,5 et 2,5 g/l.

12. Procédé selon l'une quelconque des revendications 1 à 11, **caractérisé en ce que** la lyophilisation est effectuée à des températures comprises entre -90°C et - 30°C pendant 48 heures.

13. Procédé selon l'une quelconque des revendications 1 à 12, **caractérisé en ce que** le traitement thermique d'inactivation virale est effectué à des températures comprises entre 80°C et 90°C pendant 72 heures.

14. Procédé selon l'une quelconque des revendications 1 à 13, **caractérisé en ce qu'**il comprend en outre, préalablement à l'étape d'ajout de la formulation stabilisante et solubilisante à une composition liquide de protéines cryoprécipitables, au moins une étape supplémentaire d'inactivation et/ou d'élimination des virus de ladite composition liquide par solvant-détergent et/ou par nanofiltration sur des filtres de 35 nm.

15. Procédé selon l'une quelconque des revendications 1 à 14, **caractérisé en ce qu'**il est applicable à l'ensemble des protéines cryoprécipitables.

16. Procédé selon l'une quelconque des revendications 1 à 15, **caractérisé en ce qu'**il est applicable à au moins une protéine choisie parmi le Facteur VIII, le Facteur von Willebrand, le Facteur XIII, le fibrinogène et la fibronectine.

17. Concentré d'au moins une protéine cryoprécipitable
exempte d'hydrate de carbone et de tampon tris, susceptible d'être obtenu selon le procédé selon l'une quelconque des revendications 1 à 16.

18. concentré selon la revendication 17, destiné à un usage thérapeutique.

19. Concentré selon la revendication 17 ou 18, consistant en un lyophilisat de fibrinogène remis en solution obtenu par le procédé selon la revendication 13, pour ainsi présenter une filtrabilité d'environ 2 ml/cm² sur un filtre à porosité de 0,20 ± 0,02 µm.

20. Formulation stabilisante et solubilisante, exempte d'hydrates de carbone et de tampon tris, pour les protéines cryoprécipitables destinées à être soumises à une lyophilisation et à un traitement thermique d'inactivation virale, **caractérisée en ce qu'**elle comprend un mélange d'arginine, présente en une concentration comprise entre 35 et 45 g/l, d'au moins un acide aminé hydrophobe et de citrate trisodique, présent en une concentration comprise entre 0,5 et et 12 g/l.

21. Formulation stabilisante et solubilisante selon la revendication 20, **caractérisée en ce qu'**elle est constituée dudit mélange d'arginine, d'au moins un acide aminé hydrophobe et de citrate trisodique.

## Claims

1. Method for obtaining cryoprecipitable proteins free of carbon hydrates and tris buffer, comprising a viral inactivation step by heat treatment of a lyophilisate of the said proteins **characterized in that**, before placing the proteins in lyophilisate form, it includes an initial step to add to the said proteins a stabilizing and solubilizing formulation, free of carbon hydrates and tris buffer, comprising a mixture of arginine, at least one hydrophobic amino acid and trisodium citrate.

2. The method according to claim 1, **characterized in that** the formulation is formed of the said mixture of arginine, at least one hydrophobic amino acid and trisodium citrate.

3. The method according to claim 1 or 2, **characterized in that** arginine is present at a concentration of between 25 and 50 g/l.

4. The method according to claim 3, **characterized in that** the concentration of arginine is between 35 and 45 g/l.

5. The method according to any of claims 1 to 4, **characterized in that** the trisodium citrate is present at a concentration of between 0.5 and about 12 g/l.

6. The method according to any of claims 1 to 5, **characterized in that** the hydrophobic amino acid is leucine, iso-leucine or a mixture of both.

7. The method according to claim 6, **characterized in that** the leucine, iso-leucine or the mixture thereof is present at a concentration of between 5 and 15 g/l.

8. The method according to one of claims 6 and 7, **characterized in that** the concentration of leucine or iso-leucine or the mixture thereof is between 9 and 11 g/l.

9. The method according to any of claims 1 to 8, **characterized in that** glycine and/or lysine are also added to the formulation.

10. The method according to claim 9, **characterized in that** glycine and lysine are each present at a concentration of between 1 and 5 g/l.

11. The method according to claim 10, **characterized in that** each of the concentrations of glycine and lysine is between 1.5 and 2.5 g/l.

12. The method according to any of claims 1 to 11, **characterized in that** lyophilisation is conducted at temperatures of between -40°C and -30°C for 48 hours.

13. The method according to any of claims 1 to 12, **characterized in that** the heat treatment for viral inactivation is conducted at temperatures of between 80°C and 90°C for 72 hours.

14. The method according to any of claims 1 to 13, **characterized in that**, prior to the step to add the stabilizing and solubilizing formulation to a liquid composition of cryoprecipitable proteins, it further comprises at least one additional step for inactivation and/or elimination of the viruses from the said liquid composition by solvent-detergent and/or by nanofiltration on 35 nm filters.

15. The method according to any of claims 1 to 14, **characterized in that** it can be applied to all the cryoprecipitable proteins.

16. The method according to any of claims 1 to 15, **characterized in that** it can be applied to at least one protein selected from among Factor VIII, Factor von Willebrand, Factor XIII, fibrinogen and fibronectin.

17. A concentrate of at least one cryoprecipitable protein free of carbon hydrates and tris buffer, able to be obtained using the method according to any of claims 1 to 16.

18. A concentrate according to claim 17 intended for therapeutic use.

19. A concentrate according to claim 17 or 18 consisting of a fibrinogen lyophilisate, replaced in solution, obtained using the method according to claim 13, so as to display filterability of about 2 ml/cm² through a filter of porosity 0.20 ± 0.02 µm.

20. A stabilizing and solubilizing formulation free of carbon hydrates and tris buffer, for the cryoprecipitable proteins intended to be subjected to lyophilisation and to heat treatment for viral inactivation, **characterized in that** it comprises a mixture of arginine present in a concentration of between 35 and 45 g/l, of least one hydrophobic amino acid and of trisodium citrate present in a concentration of between 0.5 and 12 g/l.

21. A stabilizing and solubilizing formulation according to claim 20, **characterized in that** it consists of the said mixture of arginine, of at least one hydrophobic amino acid and trisodium citrate.

## Patentansprüche

1. Verfahren zum Erhalt von kryopräzipitierbaren Proteinen, die frei von Kohlenhydraten und Tris-Puffer sind, das einen Schritt zur Virusinaktivierung eines Lyophilisats dieser Proteine durch Wärmebehandlung umfasst, **dadurch gekennzeichnet, dass** es vor der Umwandlung der Proteine in die Form von Lyophilisat, einen ersten Schritt zur Zugabe, zu den Proteinen, einer stabilisierenden und solubilisierenden Formulierung umfasst, die frei von Kohlenhydraten und Tris-Puffer ist, die ein Arginingemisch, mindestens eine hydrophobe Aminosäure und Trinatriumcitrat umfasst.

2. Verfahren nach Anspruch 1, **dadurch gekennzeichnet, dass** die Formulierung aus dem Arginingemisch, aus mindestens einer hydrophoben Aminosäure und aus Trinatriumcitrat besteht.

3. Verfahren nach Anspruch 1 oder 2, **dadurch gekennzeichnet, dass** das Arginin in einer Konzentration im Bereich zwischen 25 und 50 g/l vorhanden ist.

4. Verfahren nach Anspruch 3, **dadurch gekennzeichnet, dass** die Argininkonzentration im Bereich zwischen 35 und 45 g/l liegt.

5. Verfahren nach einem der Ansprüche 1 bis 4, **dadurch gekennzeichnet, dass** das Trinatriumcitrat in einer Konzentration im Bereich zwischen 0,5 und etwa 12 g/l vorhanden ist.

6. Verfahren nach einem der Ansprüche 1 bis 5, **dadurch gekennzeichnet, dass** die hydrophobe Aminosäure Leucin, Isoleucin oder ein Gemisch aus beiden ist.

7. Verfahren nach Anspruch 6, **dadurch gekennzeichnet, dass** das Leucin, Isoleucin oder ihr Gemisch in einer Konzentration im Bereich zwischen 5 und 15 g/l vorhanden ist.

8. Verfahren nach einem der Ansprüche 6 und 7, **dadurch gekennzeichnet, dass** die Konzentration an Leucin oder Isoleucin oder ihres Gemischs im Bereich zwischen 9 und 11 g/l liegt.

9. Verfahren nach einem der Ansprüche 1 bis 8, **dadurch gekennzeichnet, dass** der Formulierung ferner Glycin und/oder Lysin zugegeben werden.

10. Verfahren nach Anspruch 9, **dadurch gekennzeichnet, dass** das Glycin und das Lysin jeweils in einer Konzentration im Bereich zwischen 1 und 5 g/l vorhanden sind.

11. Verfahren nach Anspruch 10, **dadurch gekennzeichnet, dass** die Glycin- und Lysinkonzentration im Bereich zwischen 1,5 und 2,5 g/l liegt.

12. Verfahren nach einem der Ansprüche 1 bis 11, **dadurch gekennzeichnet, dass** die Lyophilisierung 48 Stunden lang bei Temperaturen im Bereich zwischen -40°C und - 30°C durchgeführt wird.

13. Verfahren nach einem der Ansprüche 1 bis 12, **dadurch gekennzeichnet, dass** die Wärmebehandlung zur Virusinaktivierung 72 Stunden lang bei Temperaturen im Bereich zwischen 80° C und 90° C durchgeführt wird.

14. Verfahren nach einem der Ansprüche 1 bis 13, **dadurch gekennzeichnet, dass** es ferner, vor dem Schritt zur Zugabe der stabilisierenden und solubilisierenden Formulierung zu einer flüssigen Zusammensetzung aus kryopräzipitierbaren Proteinen mindestens einen zusätzlichen Schritt zur Inaktivierung und/oder zur Eliminierung der Viren aus der flüssigen Zusammensetzung durch Solvent/Detergent-Verfahren und/oder durch Nanofiltration auf Filtern mit 35 nm umfasst.

15. Verfahren nach irgendeinem der Ansprüche 1 bis 14, **dadurch gekennzeichnet, dass** es auf die Gesamtheit der kryopräzipitierbaren Proteine anwendbar ist.

16. Verfahren nach einem der Ansprüche 1 bis 15, **dadurch gekennzeichnet, dass** es auf mindestens ein Protein, ausgewählt aus Faktor VIII, von-Willebrand-Faktor, Faktor XIII, Fibrinogen und Fibronectin, anwendbar ist.

17. Konzentrat aus mindestens einem kryopräzipitierbaren Protein, das frei von Kohlenhydraten und Tris-Puffer ist, das gemäß dem Verfahren nach einem der Ansprüche 1 bis 16 erhalten werden kann.

18. Konzentrat nach Anspruch 17, das für eine therapeutische Verwendung bestimmt ist.

19. Konzentrat nach Anspruch 17 oder 18, das aus einem zur ursprünglichen Konzentration gelösten Fibrinogenlyophilisat besteht, das durch das Verfahren nach Anspruch 13 erhalten wurde, damit es auf einem Filter mit einer Porosität von 0,20 ± 0,02 µm eine Filtrierbarkeit von etwa 2 ml/cm² aufweist.

20. Stabilisierende und solubilisierende Formulierung, die frei von Kohlenhydraten und Tris-Puffer ist, für die kryopräzipitierbaren Proteine, die dazu bestimmt sind, einer Lyophilisation und einer Wärmebehandlung zur Virusinaktivierung unterzogen zu werden, **dadurch gekennzeichnet, dass** sie ein Arginingemisch, das in einer Konzentration im Bereich zwischen 35 und 45 g/l vorhanden ist, mindestens eine hydrophobe Aminosäure und Trinatriumcitrat umfasst, das in einer Konzentration im Bereich zwischen 0,5 und 12 g/l vorhanden ist.

21. Stabilisierende und solubilisierende Formulierung nach Anspruch 20, **dadurch gekennzeichnet, dass** sie aus dem Arginingemisch, mindestens einer hydrophoben Aminosäure und Trinatriumcitrat besteht.
